# EUROPÄISCHE PATENTSCHRIFT

(11) **EP 1 099 677 B1**
(45) Veröffentlichungstag und Bekanntmachung des Hinweises auf die Patenterteilung: **24.03.2004**
(21) Anmeldenummer: 00121487.3
(22) Anmeldetag: 29.09.2000
(51) Int. Cl.: C07C 45/50, C07C 47/02, B01J 31/24

(54) **Verfahren zur Herstellung von Aldehyden aus Olefinen durch Hydroformylierung**
Process for the preparation of aldehydes by hydroformylation of olefins
Procédé de préparation d'aldehydes par hydroformylation d'oléfines

(30) Priorität: 12.11.1999 DE 19954721
(43) Veröffentlichungstag der Anmeldung: 16.05.2001
(73) Patentinhaber: Oxeno Olefinchemie GmbH, 45764 Marl (DE)
(72) Erfinder: Börner, Armin, Prof. Dr., 18059 Rostock (DE); Hess, Dieter, Dr., 45770 Marl (DE); Röttger, Dirk, Dr., 45657 Recklinghausen (DE); Selent, Detlef, Dr., 10318 Berlin (DE)

(56) Entgegenhaltungen:
- WO-A-98/43935
- DETLEF SELENT ET AL.: "Novel Oxyfunctionalized Phosphonite Ligands for the Hydroformylation of Isomeric n-Olefins" ANGEW. CHEM. INT. ED. , Bd. 39, Nr. 9, 2000, Seiten 1639-41, XP000941861

## Beschreibung

Die Erfindung betrifft ein Verfahren zur Herstellung von Aldehyden durch Hydroformylierung von Olefinen oder Olefingemischen in Anwesenheit eines Katalysators, bestehend aus einem Metall der VIII. Nebengruppe und einem funktionalisierten Phosphonitliganden.

Aldehyde können durch katalytische Hydroformylierung (oder Oxo-Reaktion) von um ein Kohlenstoffatom kürzeren Olefinen hergestellt werden. Die Hydrierung dieser Aldehyde ergibt Alkohole, die zum Beispiel zur Herstellung von Weichmachern oder als Detergentien genutzt werden. Die Oxidation der Aldehyde liefert Carbonsäuren, die beispielsweise zur Herstellung von Trocknungsbeschleunigern für Lacke oder als Stabilisatoren für PVC verwendet werden können.

Die Art des Katalysatorsystems und die optimalen Reaktionsbedingungen für die Hydroformylierung sind von der Reaktivität des eingesetzten Olefins abhängig. Einen kompakten Überblick über die Hydroformylierung, Beispiele für Katalysatoren und ihre Einsatzgebiete, gängige großtechnische Prozesse usw. finden sich in B. Cornils, W. A. Herrmann (Ed.), "Applied Homogeneous Catalysis with Organometallic Compounds", VCH, Weinheim, New-York, Basel, Cambridge, Tokyo, **1996,** Vol.1, S. 29-104. Die Abhängigkeit der Reaktivität der Olefine von ihrer Struktur ist unter anderem von J. Falbe, "New Syntheses with Carbon Monoxide", Springer-Verlag, Berlin, Heidelberg, New York, **1980,** S. 95 ff., beschrieben. Die unterschiedliche Reaktivität von isomeren Octenen ist ebenfalls bekannt (B. L. Haymore, A. van Hasselt, R. Beck, Annals of the New York Acad. Sci., 415 (1983), S. 159-175).

Über die unterschiedlichen Prozesse und Katalysatoren ist eine Vielzahl von Olefinen für die Oxierung zugänglich. Ein mengenmäßig bedeutender Rohstoff ist Propen, aus dem n- und i-Butyraldehyd erhalten werden.

Technische Olefingemische, die als Edukte für die Oxo-Synthese verwendet werden, enthalten oftmals Olefine der verschiedensten Strukturen mit unterschiedlichen Verzweigungsgraden, unterschiedlicher Lage der Doppelbindung im Molekül und gegebenenfalls auch unterschiedlichen C-Zahlen. Ein typisches Beispiel ist Raffinat I als Mischung der C₄-Olefine 1-Buten, 2-Buten und Isobuten. Besonders gilt dies für Olefingemische, die durch Di-, Tri- oder weitergehende Oligomerisierung von C₂-C₅-Olefinen oder anderen leicht zugänglichen höheren Olefinen bzw. durch Cooligomerisierung von Olefinen entstanden sind. Als Beispiele für technische Olefingemische, die durch Hydroformylierung zu den entsprechenden Aldehydgemischen umgesetzt werden können, seien Tri- und Tetrapropene sowie Di-, Triund Tetrabutene genannt.

Die Produkte der Hydroformylierung sind durch die Struktur der Einsatzolefine, das Katalysatorsystem und die Reaktionsbedingungen bestimmt. Unter Bedingungen, bei denen es zu keiner Verschiebung der Doppelbindung im Olefin kommt, im Folgenden als nicht-isomerisierende Bedingungen bezeichnet, wird die Formylgruppe an der Stelle im Molekül eingeführt, an der sich die Doppelbindung befand, wobei wiederum zwei unterschiedliche Produkte entstehen können. So kann beispielsweise bei der Hydroformylierung von 1-Penten Hexanal und 2-Methylpentanal gebildet werden. Bei der Hydroformylierung unter isomerisierenden Bedingungen, bei der neben der eigentlichen Oxierung auch eine Verschiebung der Doppelbindung im Olefin stattfindet, würde bei der Hydroformylierung von 1-Penten zusätzlich 2-Ethylbutanal als Produkt zu erwarten sein.

Sind als Folgeprodukt der Oxoaldehyde Alkohole für die Herstellung von Detergentien und Weichmacher angestrebt, so sollen durch die Oxoreaktion möglichst linerare Aldehyde hergestellt werden. Die hieraus erhältlichen linearen Alkohole können zu den entsprechenden Phthalaten umgesetzt werden; diese Phthalate besitzen besonders vorteilhafte Eigenschaften wie z. B. eine niedrige Viskosität.

Die oben genannten technischen Olefingemische enthalten oftmals nur geringe Anteile an Olefinen mit endständiger Doppelbindung. Um aus ihnen Produkte herzustellen, in denen mehr terminal oxiertes Olefin vorliegt, als im ursprünglichen Olefingemisch Olefine mit endständiger Doppelbindung, muß unter isomerisierenden Bedingungen hydroformyliert werden. Geeignete Verfahren dafür sind beispielsweise Hochdruck-Hydroformylierungen mit Kobaltkatalysatoren. Diese Verfahren haben jedoch den Nachteil, daß relativ viel Nebenprodukte, zum Beispiel Alkane, Acetale oder Ether gebildet werden.

Bei der Verwendung von Rhodiumkomplexen als Katalysator für Oxoreaktionen ist der Ligand für die Produktzusammensetzung der Aldehyde mitbestimmend. Rhodiumcarbonyle ohne Phosphor-, Arsen- oder Stickstoffhaltige Liganden (unmodifizierter Rhodiumkatalysator) katalysieren die Hydroformylierung von Olefinen mit endständigen und innenständigen Doppelbindungen, wobei die Olefine auch verzweigt sein können, zu Aldeyhden mit einem hohen Verzweigungsgrad. Der Anteil an terminal oxiertem Olefin ist, im Vergleich zum Kobalt-oxierten Produkt, deutlich geringer.

Mit ligandmodifizierten Rhodium-Katalysatoren, bestehend aus Rhodium und Triorganophosphin, z. B. Triphenylphosphin werden α-Olefine mit hoher Selektivität terminal hydroformyliert. Eine Isomerisierung der Doppelbindungen und/oder die Hydroformylierung der innenständigen Doppelbindungen tritt kaum auf. Mit Hilfe von Katalysatorsystemen, die sterisch anspruchsvolle Phosphitliganden enthalten, wird zwar eine isomerisierende Hydroformylierung erreicht, allerdings sind die Ausbeuten an endständig oxierten Olefinen, die innenständige Doppelbindungen an Verzweigungsstellen enthalten, nicht zufriedenstellend. Eine Übersicht über den Einfluß von Liganden auf die Aktivität und Selektivität bei der Hydroformylierung findet sich im oben zitierten Buch von B. Cornils und W. A. Herrmann.
Verglichen mit Phosphin- oder Phosphitliganden finden sich in der Fachliteratur nur wenige Publikationen zum Einsatz von Phosphonigsäurediestern (im Folgenden Phosphonite genannt) als Liganden in Oxierungsreaktionen. In WO 98/43935 werden Katalysatorsysteme, bestehend aus Rhodium, einem Triorganophosphonit-Liganden oder einen zweizähnigen Phosphonitliganden für die Hydroformylierung von acyclischen, cyclischen Olefinen bzw. Olefingemischen beschrieben.

JP-OS Hei 9-268152 offenbart die Verwendung von acyclischen Phosphonitliganden für Hydroformylierungsreaktionen. Diese acyclischen Liganden sind nur aufwendig herzustellen und daher für einen großtechnischen Prozeß ungeeignet.

JP-OS 9-255610 beschreibt analog die Verwendung von cyclischen Phosphoniten. Hier bildet ein Bisarylsystem mit je einem Phosphor- und Sauerstoffatom ein Phenanthren-ähnliches Gerüst, an das über ein weiteres Sauerstoffatom ein gegebenenfalls substituierter Arylrest gebunden ist. Systeme dieser Art sind in Bezug auf die Selektivität von Hydroformylierungsreaktionen noch verbesserungsfähig.

Aufgabe der vorliegenden Erfindung war es daher, ein Verfahren zur Hydroformylierung von Olefinen unter Verwendung von Phosphonitliganden bereitzustellen, mit dem verzweigte, unverzweigte, end- oder innenständige Olefine mit hohen Ausbeuten und Selektivitäten terminal oxiert werden können, d.h. möglichst lineare Aldehyde hergestellt werden können.

Es wurde überraschend gefunden, daß Hydroformylierungen von Olefinen unter Katalyse von Metallkomplexen, bestehend aus einem Metall der 8. Nebengruppe und Phosponiten, Arsenoiten und Stibenoiten mit hohen Ausbeuten und Selektivitäten zu linearen, endständig oxierten Olefinen führt.
Gegenstand der vorliegenden Erfindung ist daher ein Verfahren zur katalytischen Hydroformylierung von Olefinen mit 3 bis 24 Kohlenstoffatomen, wobei als Katalysator ein Metall der 8. Nebengruppe des Periodensystems in Gegenwart eines Liganden der Formel I 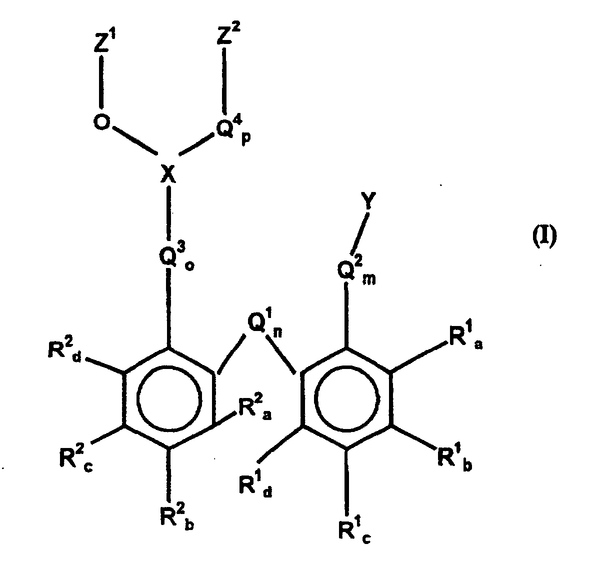 mit
- X =: As, Sb, P,
- R¹_{a-d}, R²_{a-d} =: H, aliphatischer oder aromatischer Kohlenwasserstoffrest, aliphatische oder aromatische Alkoxygruppe, jeweils mit 1 bis 25 Kohlenstoffatomen, wobei R¹_{a-d} und R²_{a-d} jeweils gleich oder verschieden sein können,
- Q¹, Q², Q³, Q⁴=: O, S, NR⁷, CR⁷R⁸, wobei R⁷ und R⁸ gleich oder verschieden und eine der Bedeutungen von R¹ₐ besitzen können, mit der Maßgabe, daß entweder Q³ oder Q⁴ O, S, NR⁷ bedeuten,
- n, m, o, p =: 0 oder 1, mit der Maßgabe, daß entweder o oder p 1 ist,
- Y =: -O-R⁵, -COOR⁵, -COOM, -SR⁵, -NR⁵R⁶, -N=CR⁵R⁶, -COR⁵, -CONR⁵R⁶, -F, -Cl, -Br, -I
wobei R⁵ und R⁶ gleich oder verschieden sein können und H, einen aliphatischen oder aromatischen Kohlenwasserstoffrest mit 1 bis 25 Kohlenstoffatomen bezeichnen und M = H, Li, Na, K oder NH₄ bedeuten und
- Z¹, Z² =: substituierter oder unsubstituierter, aliphatischer oder aromatischer Kohlenwasserstoffrest mit 1 bis 75 Kohlenstoffatomen, wobei Z¹ und Z² eine kovalente Verknüpfung aufweisen können,
eingesetzt wird.

In besonderen Ausführungsformen der vorliegenden Erfindung können auch Liganden der Formeln II, III oder IV eingesetzt werden:

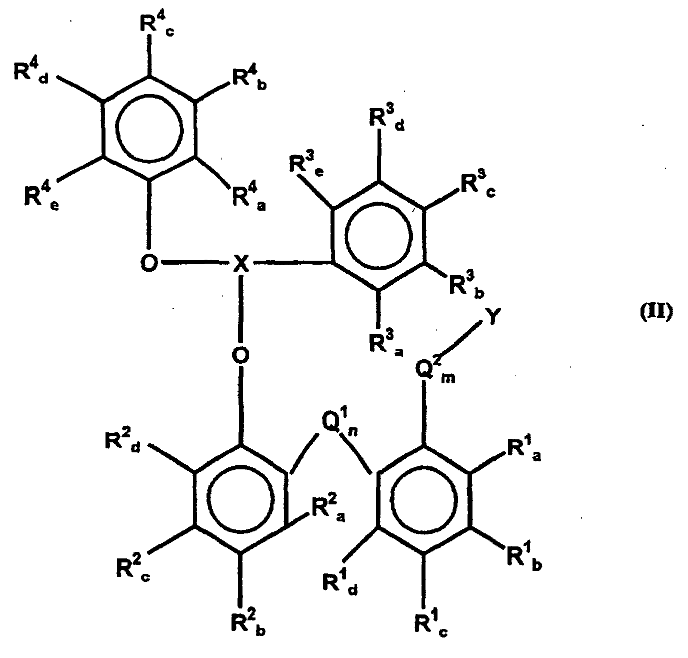

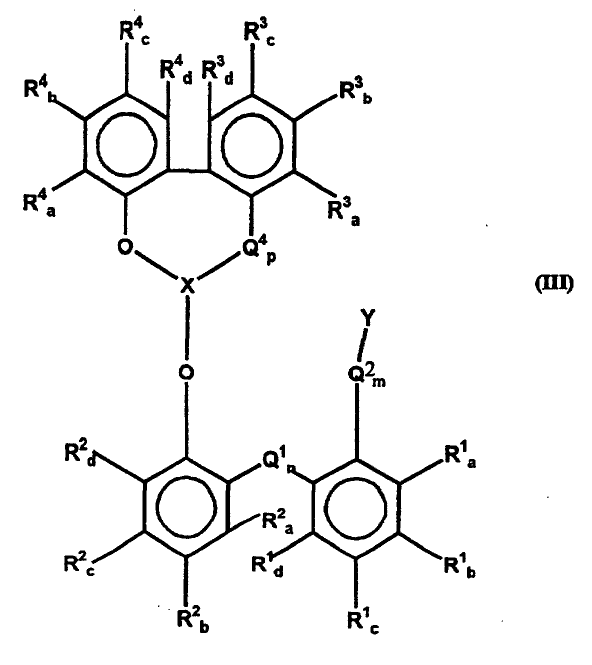

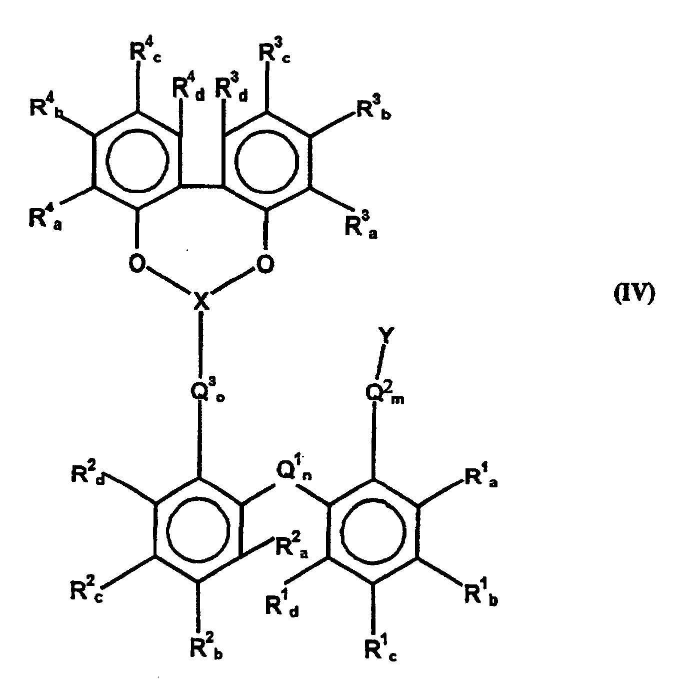

Die Reste R¹_{a-d}, R²_{a-d}, R³ₐ₋ₑ und R⁴ₐ₋ₑ bedeuten in diesen Formeln jeweils H, aliphatischer oder aromatischer Kohlenwasserstoffrest, eine aliphatische oder aromatische Alkoxygruppe, jeweils mit 1 bis 25 Kohlenstoffatomen, wobei R¹_{a-d}, R²_{a-d}, R³ₐ₋ₑ, R⁴ₐ₋ₑ jeweils gleich oder verschieden sein können. So kann z. B. R¹ₐ eine Methyl- und R¹_{b} eine Methoxygruppe bezeichnen; analoges gilt für die Reste R²_{a-d}, R³ₐ₋ₑ, R⁴ₐ₋ₑ.
Q¹ und Q² stehen jeweils für O, S, NR⁷ einen Methylenrest CR⁷R⁸, wobei R⁷ und R⁸ gleich oder verschieden und eine der Bedeutungen von R¹ₐ besitzen können. Q³ und Q⁴ stehen jeweils für einen Methylenrest CR⁷R⁸, wobei R⁷ und R⁸ gleich oder verschieden und eine Bedeutung von R¹ₐ besitzen können. Die Indices n, m, o und p stehen jeweils für 0 oder 1, ggf. mit der Maßgabe, daß entweder o oder p 1 ist.

Y steht für -O-R⁵, -COOR⁵, -COOM, -SR⁵, -NR⁵R⁶, -N=CR⁵R⁶, -COR⁵, -CONR⁵R⁶, -F, -Cl, -Br, -I wobei R⁵ und R⁶ gleich oder verschieden sein können und H, einen aliphatischen oder aromatischen Kohlenwasserstoffrest mit 1 bis 25 Kohlenstoffatomen bezeichnen und M = H, Li, Na, K oder NH₄ bedeuten.

X bezeichnet wie in Formel I die Elemente As, Sb oder P.

Liganden, die im erfindungsgemäßen Verfahren eingesetzt werden können, sind beispielsweise:

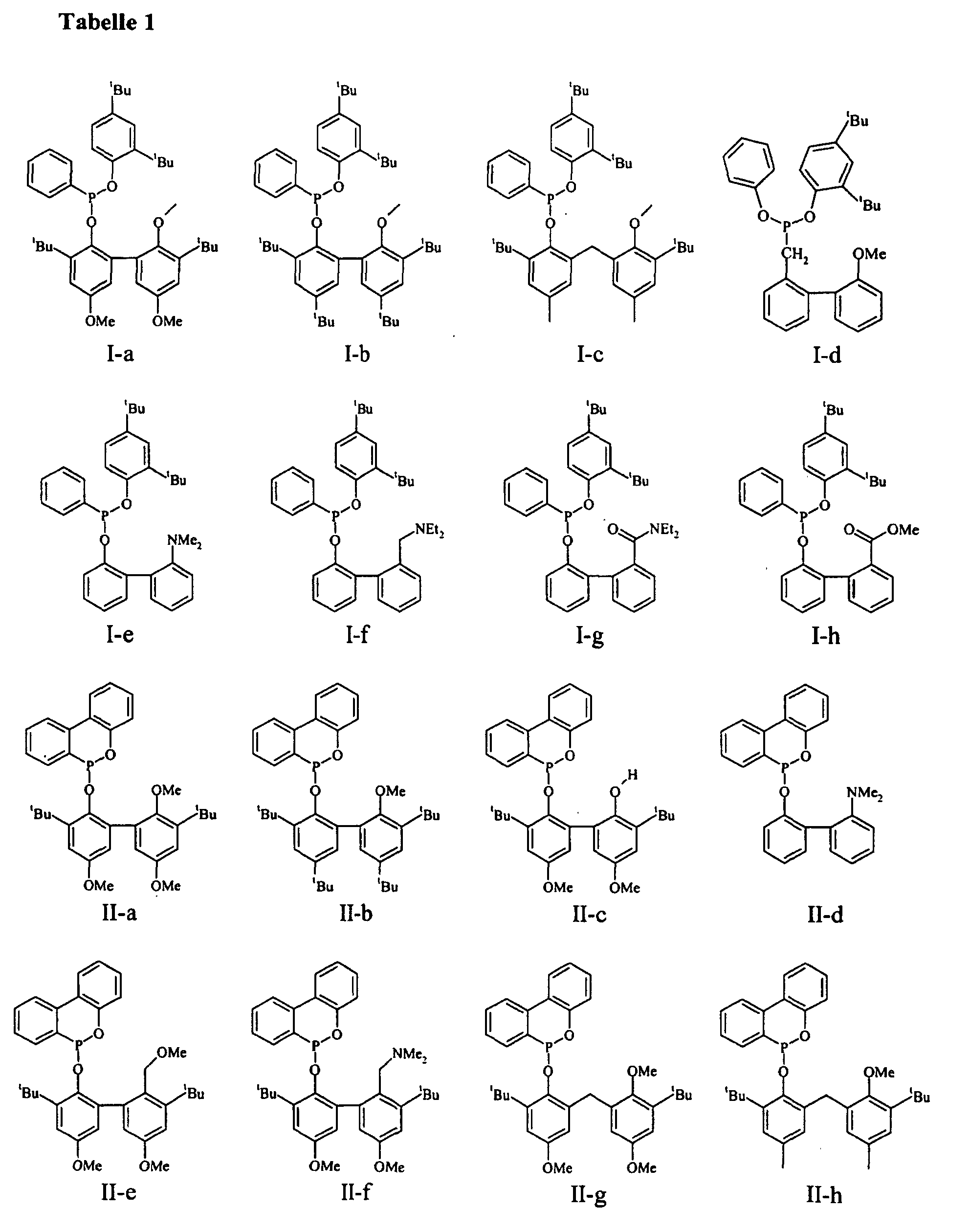

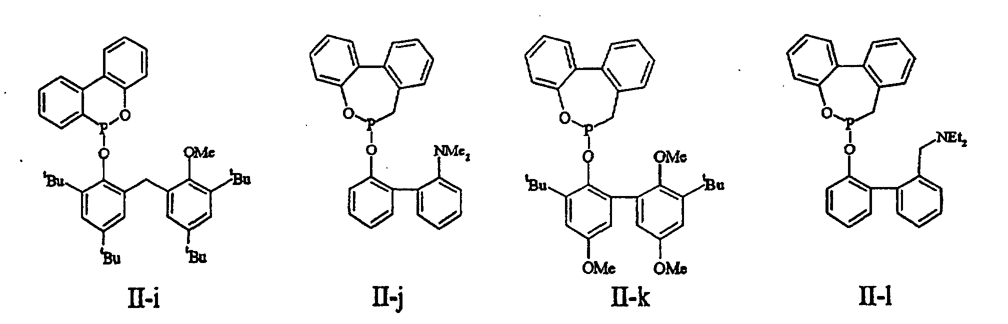

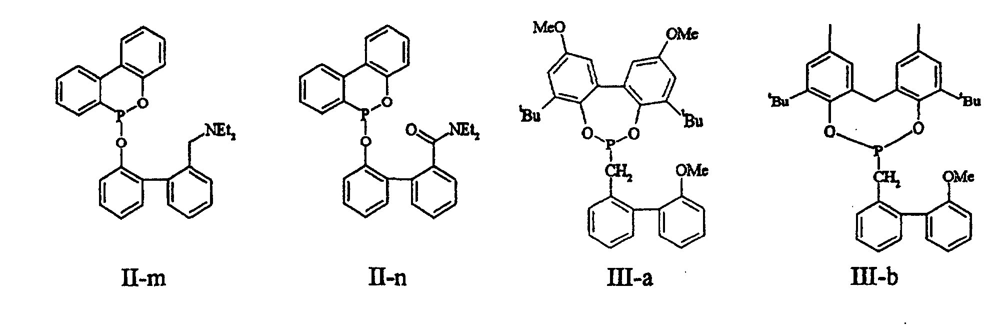

Die im erfindungsgemäßen Verfahren eingesetzten Liganden der Formel I, II, III oder IV werden im Folgenden jeweils als heterofunktionalisierte Phosphonite, Arsenonite oder Stibonite bezeichnet. Liganden dieser Art können mit Metallatomen der 8. Nebengruppe des Periodensystems hemilabile Komplexe bilden.
Unter diesen heterofunktionalisierten Phosphoniten, Arsenoniten oder Stiboniten werden Verbindungen mit einem Atom der fünften Hauptgruppe des Periodensystems (P, As, Sb) verstanden, das ein freies Elektronenpaar und zwei Einfachbindungen zu jeweils einem Sauerstoffatom und eine Einfachbindung zu einem Kohlenstoffatom besitzt. Die allgemeinen Formeln I bis IV und die Beispiele der Tabelle 1 zeigen mögliche Liganden für das erfindungsgemäße Verfahren.

Die Liganden enhalten neben dem Atom der 5. Hauptgruppe mindestens ein weiteres Heteroatom mit mindestens einem freien Elektronenpaar. Das Atom der fünften Hauptgruppe und das weitere Heteroatom sind im Ligand derart angeordnet, daß ein Metallatom intramolekular an diesen beiden Atomen gleichzeitig koordiniert sein kann. Die trifft zum Beispiel zu, wenn ein Phosphoratom, ein Heteroatom und die dazwischenliegenden Atome mit dem koordinierten Metallatom einen 4 bis 15-, bevorzugt 8 bis 12-gliedrigen Ring, bilden können. Dieser Ring kann in den allgemeinen Formel I bis IV über das Metall der 8. Nebengruppe, das Atom X und den Substituenten Q²-Y gebildet werden.

Die in dem Rest Y enthaltenen Heteroatome können Sauerstoff, Schwefel, Stickstoff, Fluor, Chlor, Brom oder Jod sein. Die Heteroatome können in funktionellen Gruppen wie z.B. Ethern, Thioethern und tertiären Aminen enthalten sein und/oder Teil einer Kette oder eines Ringes sein. Es ist auch möglich, daß die Liganden mehr als ein Heteroatom enthalten, das diesen Forderungen entspricht. Die erfindungsgemäß eingesetzten Liganden sollten eine koordinative Bindung zwischen Heteroatom und Metall aufweisen, die eine geringere Stärke besitzt, als die zwischen dem Atom der fünften Hauptgruppe, d.h. P, As, Sb und dem Metall.

In der Fachliteratur werden Liganden, die neben einer starken Wechselwirkung zu einem Metall eine zweite, aber deutlich schwächere (labile) Wechselwirkung aufweisen, oft als hemilabile Liganden bezeichnet (Übersichtsartikel: A. Bader, E. Linder, Coord. Chem. Rev. **1991,** 108, 27-110; C. S. Slone, D. A. Weinberger, C. A. Mirkin, Prog. Inorg. Chem. **1999,** 48, 233). Für einige Literaturbeispiele konnte anhand von Röntgenstrukturen die zweite, schwächere Wechselwirkung des Liganden mit dem Metall nachgewiesen werden. Für die vorliegenden heterofunktionalisierten Liganden ist das Koordinationsverhalten nicht bekannt, es kann aber aus sterischen Überlegungen geschlossen werden, daß jeweils eine Koordination des Metallatoms über z. B. ein Phosphoratom und ein zusätzliches Heteroatom möglich ist.

Die im erfindungsgemäßen Verfahren eingesetzten Liganden der allgemeinen Formeln I, II, III oder IV bilden vermutlich eine hemilabile Bindung über die Gruppe mit der Bezeichnung Y aus. Der Bisarylsubstituent mit der funktionellen Gruppe Y stellt ein wichtiges Merkmal der im erfindungsgemäßen Verfahren eingesetzten Liganden dar, da mit diesen Liganden hemilabile Bindungen zum Zentralmetall des Katalysatorkomplexes ausgebildet werden können.

Das erfindungsgemäße Verfahren kann mit verschiedenen Katalysatoren und/oder Liganden durchgeführt werden.

Als katalytisch aktives Metall kommen die Metalle der 8. Nebengruppe des Periodensystems der Elemente in Frage, wie Rhodium, Kobalt, Platin oder Ruthenium.

Der aktive Katalysatorkomplex für die Hydroformylierung wird dabei aus einem Salz oder einer Verbindung des Metalls (Katalysatorvorläufer), dem Liganden und Synthesegas gebildet, zweckmäßig geschieht dies in situ während der Hydroformylierung. Übliche Katalysatorvorläufer sind beispielsweise Octanoate oder Acetylacetonate. Das molare Verhältnis zwischen Metall und Ligand liegt bei 1/1 bis 1/1000, bevorzugt zwischen 1/1 und 1/50. Die Konzentration des Metalls im Reaktionsgemisch liegt im Bereich von 1 ppm bis 1000 ppm, vorzugsweise im Bereich 5 ppm bis 300 ppm. Die Reaktionstemperaturen des erfindungsgemäßen Verfahrens liegen zwischen 60 °C und 180 °C, vorzugsweise zwischen 90 °C und 150 °C, die Drücke betragen 1-300 bar, vorzugsweise 15-60 bar.
Der Katalysator, d. h. Metall und Ligand ist homogen im Hydroformylierungsgemisch, bestehend aus Edukt (Olefin) und Produkt (Aldehyde, Alkohole, Hochsieder), gelöst. Optional kann zusätzlich ein Lösungsmittel, z. B. Toluol, Texanol, hochsiedende Rückstände aus dem Oxoprozeß oder Phthalate wie Di(2-ethyl-hexyl)phthalat, verwendet werden.

Die Edukte für eine Hydroformylierung gemäß dem Verfahren der Erfindung sind Olefine oder Gemische von Olefinen, insbesondere Monoolefine mit 3 bis 24, bevorzugt 4 bis 16, besonders bevorzugt 3 bis 12 Kohlenstoffatomen mit end- oder innenständigen C-C-Doppelbindungen, wie z. B. 1- oder 2-Penten, 2-Methylbuten-1, 2-Methylbuten-2, 3-Methylbuten-1, 1-,2- oder 3-Hexen, das bei der Dimerisierung von Propen anfallende C₆-Olefingemisch (Dipropen), Heptene, 2- oder 3-Methyl-1-hexen, Octene, 2-Methylheptene, 3-Methylheptene, 5-Methylhepten-2, 6-Methylhepten-2, 2-Ethylhexen-1, das bei der Dimerisierung von Butenen anfallende isomere C₈-Olefingemisch (Dibuten), Nonene, 2- oder 3-Methyloctene, das bei der Trimerisierung von Propen anfallence C₉-Olefingemisch (Tripropen), Decene, 2-Ethyl-1-octen, Dodecene, das bei der Tetramerisierung von Propen oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische, gegebenenfalls nach destillativer Trennung in Fraktionen mit gleicher oder ähnlicher Kettenlänge. Ebenfalls können Olefine oder Olefingemische, die durch Fischer-Tropsch-Synthese erzeugt worden sind, sowie Olefine, die durch Oligomerisierung von Ethen erhalten wurden oder Olefine, die über Methathesereaktionen zugänglich sind, eingesetzt werden. Bevorzugte Edukte sind C₄-, C₈-, C₉-, C₁₂- oder C₁₆-Olefingemische.

Mit dem erfindungsgemäßen Verfahren können unter Verwendung der heterofunktionalisierten Liganden α-Olefine, verzweigte, innenständige und innenständig verzweigte Olefine mit hohen Raum-Zeit-Ausbeuten hydroformyliert werden. Bemerkenswert ist dabei die hohe Ausbeute an endständig hydroformyliertem Olefin, selbst wenn im Edukt nur ein geringer Anteil an Olefinen mit endständiger Doppelbindung vorhanden waren.

Die folgenden Beispiele sollen die Erfindung weiter erläutern, nicht aber ihren Anwendungsbereich beschränken, der sich aus den Patentansprüchen ergibt.

### Beispiele 1 - 17 (Oxierung von Octenen)

In einen 200 ml-Autoklaven wurden unter Schutzgas 30 ml reines trockenes Toluol, 1,875 mg (0,00604 mmol) [acacRh(COD)] (Rhodiumcyclooctadienylacetylacetonat), gelöst in 10 ml Toluol, und 0,00604 bzw. 0,01208 mmol des jeweiligen Liganden, gelöst in 1 ml Toluol, gefüllt. In eine Druckpipette über dem Reaktor gab man 15 ml (10,62 g, 94,63 mmol) Octengemisch (Zusammensetzung siehe Tabelle 2). Reaktor und Druckpipette wurden über einen der Druckregelstrecke parallel geschalteten Bypass mit 33 bar CO/H₂ (1/1 Synthesegas) beaufschlagt und der Reaktorinhalt unter Rühren mit einem Begasungsrührer mit 1500 Upm auf die Reaktionstemperatur gebracht. Nach Druckerhöhung auf 45 bis 47 bar wurde das Olefingemisch aus der Druckpipette in den Reaktor gedrückt. Die vorgesehene Temperatur und der Solldruck wurden eingestellt. Der Bypass wurde geschlossen und der Druck über die gesamte Reaktionszeit mit einem Druckregler konstant (50 bar bei den Beispielen 1 - 11) gehalten. Der Versuch wurde unter Zwangskühlung beendet, wenn die mit einem Gasflussmesser beobachteten Gasverbrauchsraten unter 2 ml/min abfielen. Die Reaktionslösung wurde unter Schutzgas entnommen und gaschromatographisch analysiert.

Für die in der Tabelle 3 zusammengefaßten Beispiele 1 - 11 wurden zwei Mischungen (A und B) von Octenen eingesetzt (Zusammensetzung siehe Tabelle 2). Die Nummerierung der eingesetzten Phosphonitliganden (Ia, Ib, IIa, IIb, IIc) entspricht der in Tabelle 1.

**Tabelle 2**

| | A (Gew.-%) | B (Gew.-%) |
|---|---|---|
| n-Octen-1 | 9,8 | 3,4 |
| cis+trans-Octen-2 | 70,0 | 49,8 |
| cis+trans-Octen-3 | 15,5 | 30,0 |
| cis+trans-Octen-4 | 4,7 | 16,8 |

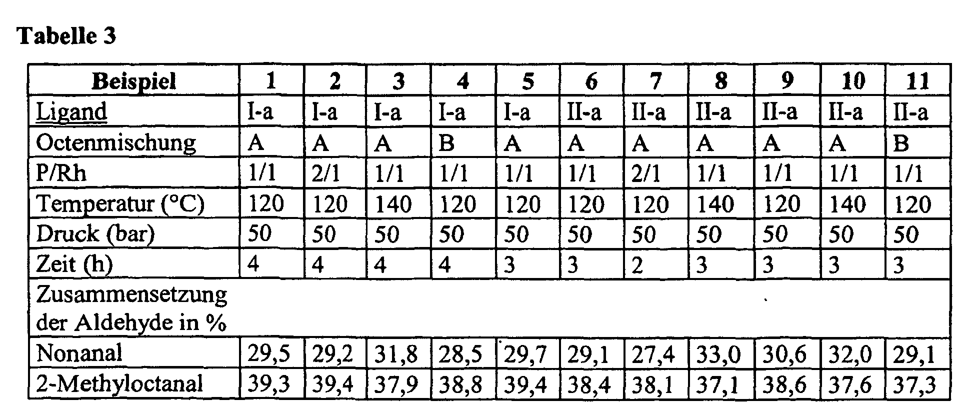

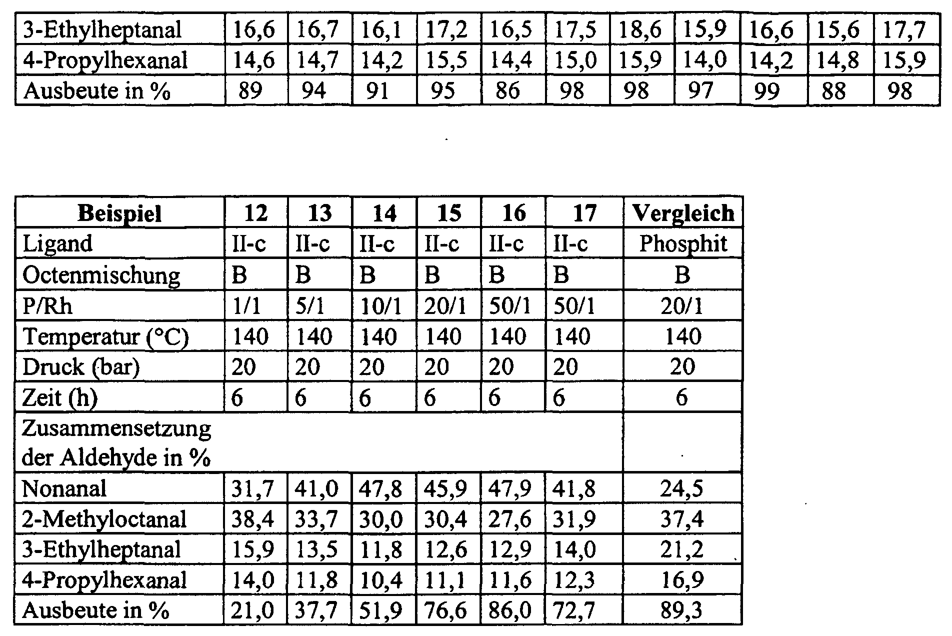

### Bemerkung zu Beispiel 17:

Dreifache Olefinkonzentration, inverse Versuchsdurchführung: Olefin vorgelegt und temperiert, Rh und Ligand in Toluol gelöst, aus Pipette zugegeben.

### Vergleichsbeispiel

Es wurde unter den Bedingungen von Beispiel 12 oxiert, an Stelle des heterofunktionalisierten Phosphonits wurde aber ein Phosphitligand (Tris[2.4-ditertbutylphenyl]phosphit) verwendet. Der Anteil an Nonanal an der Gesamtaldehydmenge betrug 24,5%.

### Beispiele 18 21 (Oxierung von Di-n-buten)

Die Versuche 18 - 21 wurden analog den Versuchen 1 - 17 durchgeführt. Als Olefin wurde dimerisiertes n-Buten (Di-n-buten) eingesetzt. Der Anteil an Olefin mit endständiger Doppelbindung (im Wesentlichen Octen-1, 3-Methylhepten-1, 5-Methylhepten-1, 2-Ethylhexen-1, 3.4-Dimethylhexen-1, 2-Ethyl-3-Methylpenten-1) beträgt unter 5%.
Die Versuche wurden nach jeweils 8 h beendet.

**Tabelle 4**

| **Beispiel** | **18** | **19** | **20** | **21** |
|---|---|---|---|---|
| Temperatur (°C) | 140 | 140 | 150 | 130 |
| Druck (bar) | 20 | 20 | 30 | 30 |
| Zeit (h) | 8 | 8 | 8 | 8 |
| Rh-Konzentration (ppm) | 20 | 150 | 150 | 20 |
| P/Rh | 10 | 10 | 10 | 10 |
| Olefin | Di-n-buten | Di-n-buten | Di-n-buten | Di-n-buten |
| Ligand | II-a | II-a | II-a | II-a |
| Umsatz (%) | 19,5 | 30,2 | 52,4 | 29,0 |
| n % * | 41,3 | 40,3 | 38,8 | 35,4 |

| | | | | |
|---|---|---|---|---|
| * Anteil der durch endständige Oxierung entstandenen Aldehyde (im Wesentlichen Nonanal, 4-Methyloctanal, 3-Ethylheptanal, 6-Methyloctanal, 4,5-Dimethylheptanal) an der Gesamtmenge an gebildeten Aldehyden | | | | |

In den Beispielen 18 - 21 zeigt sich, daß mit den neuen Katalysatorsystemen auch bei der Oxierung von technischen Olefingemischen, die hauptsächlich verzweigte Olefine mit innenständigen Doppelbindungen enthalten, ein hoher Anteil an endständig hydroformyliertem Produkt erhalten wird.

## Patentansprüche

1. Verfahren zur katalytischen Hydroformylierung von Olefinen mit 3 bis 24 Kohlenstoffatomen.
**dadurch gekennzeichnet.**
**daß** als Katalysator ein Metall der 8. Nebengruppe des Periodensystems in Gegenwart eines Liganden der Formel I 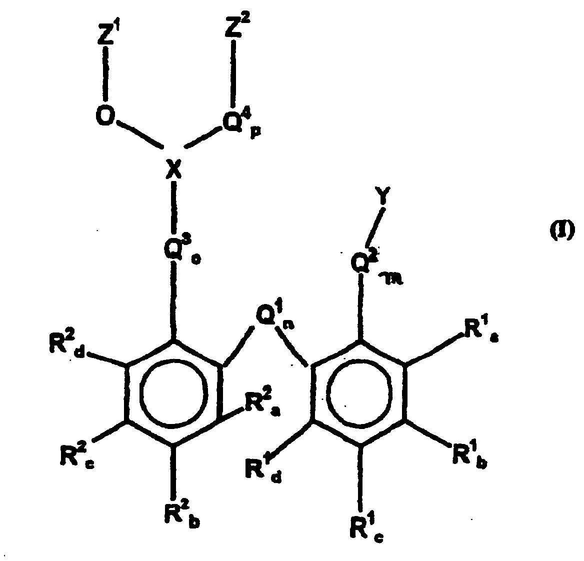 mit
X = As, Sb, P,
R¹_{a-d},R²_{a-d} = H, aliphatischer oder aromatischer Kohlenwasserstoffrest, aliphatische oder aromatische Alkoxygruppe, jeweils mit 1 bis 25 Kohlenstoffatomen, wobei R¹_{a-d} und R²_{a-d} jeweils gleich oder verschieden sein können,
Q¹,Q²,Q³,Q⁴ = O, S, NR⁷, CR⁷R⁸, wobei R⁷ und R⁸ gleich oder verschieden und eine der Bedeutungen von R¹ₐ besitzen können, mit der Maßgabe, daß entweder Q³ oder Q⁴ O, S, NR⁷ bedeuten,
n, m, o, p = 0 oder 1, mit der Maßgabe, daß entweder o oder p 1 ist,
Y = -O-R⁵, -COOR⁵, -COOM, -SR⁵, -NR⁵R⁶, -N=CR⁵R⁶, -COR⁵, -CONR⁵R⁶, -F, -Cl, -Br, -I wobei R⁵ und R⁶ gleich oder verschieden sein können und H. einen aliphatischen oder aromatischen Kohlenwasserstoffrest mit 1 bis 25 Kohlenstoffatomen bezeichnen und M = H, Li, Na, K oder NH4 bedeuten und
Z¹, Z² = substituierter oder unsubstituierter, aliphatischer oder aromatischer Kohlenwasserstoffrest mit 1 bis 75 Kohlenstoffatomen. wobei Z¹ und Z² eine kovalente Verknüpfung aufweisen können.
eingesetzt wird.

2. Verfahren nach Anspruch 1,
**dadurch gekennzeichnet,**
**daß** die Hydroformylierung in Gegenwart eines Liganden der Formel II 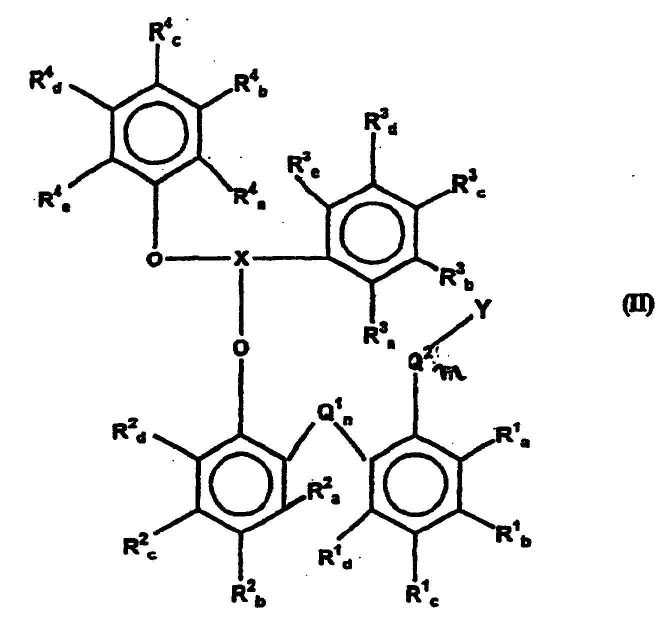 mit
X = As, Sb, P,
R¹_{a-d}, R²_{a-d}, R³_{a-c}, R⁴_{a-c}= H, aliphatischer oder aromatischer Kohlenwasserstoffrest, aliphatische oder aromatische Alkoxygruppe, jeweils mit 1 bis 25 Kohlenstoffatomen, wobei R¹_{a-d}, R²_{a-d}, R³ₐ₋ₑ und R⁴ₐ₋ₑ jeweils gleich oder verschieden sein können,
Q¹, Q², = O, S, NR⁷, CR⁷R⁸, wobei R⁷ und R⁸ gleich oder verschieden und eine der Bedeutungen von R¹ₐ besitzen können,
n, m, = 0 oder 1, und
Y = -O-R⁵, -COOR⁵, -COOM. -SR⁵. -NR⁵R⁶, -N=CR⁵R⁶, -COR⁵, -CONR⁵R⁶, -F, -Cl, -Br, -I
wobei R⁵ und R⁶ gleich oder verschieden sein können und H, einen aliphatischen oder aromatischen Kohlenwasserstoffrest mit 1 bis 25 Kohlenstoffatomen bezeichnen und M = H, Li, Na, K oder NH₄ bedeuten,
durchgeführt wird.

3. Verfahren nach Anspruch 1,
**dadurch gekennzeichnet,**
**daß** die Hydroformylierung in Gegenwart eines Uganden der Formel III 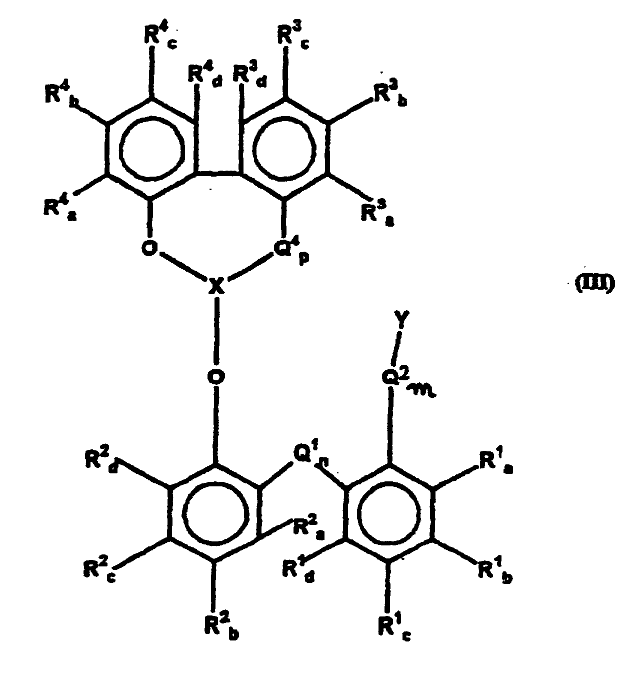 mit
X = As, Sb, P,
R¹_{a-d}, R²_{a-d}, R³_{a-d}, R⁴_{a-d}, = H, aliphatischer oder aromatischer Kohlenwasserstoffrest, aliphatische oder aromatische Alkoxygruppe, jeweils mit 1 bis 25 Kohlenstoffatomen, wobei R¹_{a-d}, R²_{a-d}, R³_{a-d}, R⁴_{a-d} jeweils gleich oder verschieden sein können,
Q¹, Q², = O, S, NR⁷, CR⁷R⁸, wobei R⁷ und R⁸ gleich oder verschieden und eine der Bedeutungen von R¹ₐ besitzen können.
Q⁴ = CR⁷R⁸, wobei R⁷ und R⁸ gleich oder verschieden sein können und eine der Bedeutungen von R¹ₐ besitzen können.
n, m, p = 0 oder 1,
Y = -O-R⁵, -COOR⁵, -COOM, -SR⁵, -NR⁵R⁶, -N=CR⁵R⁶, -COR⁵, -CONR⁵R⁶, -F, -Cl, -Br, -I wobei R⁵ und R⁶ gleich oder verschieden sein können und H, einen aliphatischen oder aromatischen Kohlenwasserstoffrest mit 1 bis 25 Kohlenstoffatomen bezeichnen und M = H, Li, Na, K oder NH₄ bedeuten.
durchgeführt wird.

4. Verfahren nach Anspruch 1,
**dadurch gekennzeichnet,**
**daß** die Hydroformylierung in Gegenwart eines Liganden der Formel IV 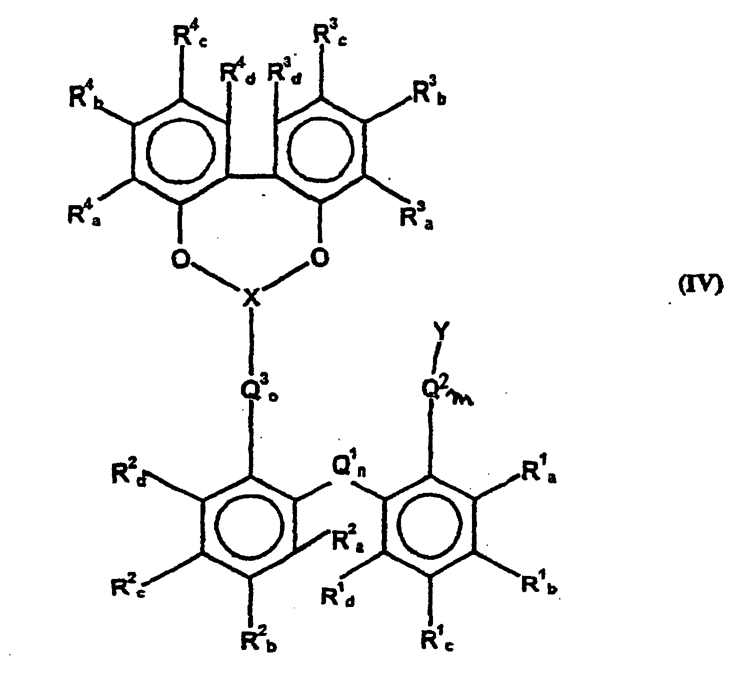 mit
X= As, Sb, P,
R¹_{a-d}, R²_{a-d}, R³_{a-d}, R⁴_{a-d}= H, aliphatischer oder aromatischer Kohlenwasserstoffrest, aliphatische oder aromatische Alkoxygruppe, jeweils mit 1 bis 25 Kohlenstoffatomen, wobei R¹_{a-d}, R²_{a-d}, R³_{a-d} und R⁴_{a-d} jeweils gleich oder verschieden sein können,
Q¹, Q², = O, S, NR⁷, CR⁷R⁸, wobei R⁷ und R⁸ gleich oder verschieden und eine der Bedeutungen von R¹ₐ besitzen können,
Q³ = CR⁷R⁸, wobei R⁷ und R⁸ gleich oder verschieden sein können und eine der Bedeutungen von R¹ₐ besitzen können.
n, m, o, = 0 oder 1.
Y = -O-R⁵, -COOR⁵, -COOM, -SR⁵, -NR⁵R⁶, -N=CR⁵R⁶, -COR5, -CONR⁵R⁶, -F, -Cl, -Br, -I wobei R5 und R⁶ gleich oder verschieden sein können und H, einen aliphatischen oder aromatischen Kohlenwasserstoffrest mit 1 bis 25 Kohlenstoffatomen bezeichnen und M = H, Li, Na, K oder NH₄ bedeuten,
durchgeführt wird.

5. Verfahren nach einem der Ansprüche 1 bis 4,
**dadurch gekennzeichnet,**
**daß** als Metall der 8. Nebengruppe des Periodensystems Kobalt oder Rhodium eingesetzt wird.

6. Verfahren nach einem der Ansprüche 1 bis 5,
**dadurch gekennzeichnet,**
**daß** Olefine mit 3 bis 8 Kohlenstoffatomen eingesetzt werden.

## Claims

1. A process for the catalytic hydroformylation of olefins having from 3 to 24 carbon atoms,
**characterized in that** the catalyst used is a metal of transition group VIII of the Periodic Table in the presence of a ligand of the formula I 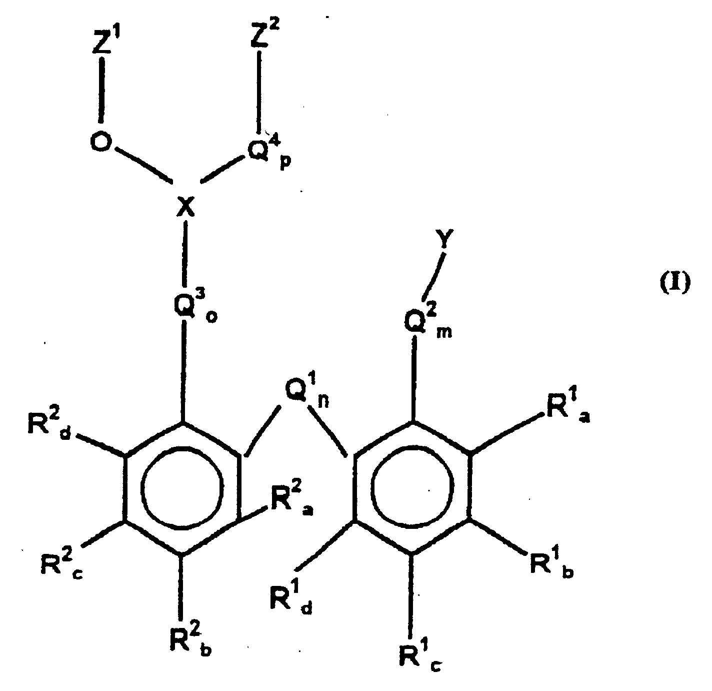 where
X = As, Sb, P,
R¹_{a-d}, R²_{a-d} = H, aliphatic or aromatic hydrocarbon radicals, aliphatic or aromatic alkoxy groups, each having from 1 to 25 carbon atoms, where R¹_{a-d} and R²_{a-d} may in each case be identical or different,
Q¹, Q², Q³, Q⁴ = O, S, NR⁷, CR⁷R⁸, where R⁷ and R⁸ may be identical or different and may be as defined for R¹ₐ, with the proviso that either Q³ or Q⁴ is O, S, NR⁷,
n, m, o, p = 0 or 1, with the proviso that either o or p is 1,
Y = -O-R⁵, -COOR⁵, -COOM, -SR⁵, -NR⁵R⁶, -N=CR⁵R⁶, -COR⁵, -CONR⁵R⁶, -F, -Cl, -Br, -I, where R⁵ and R⁶ may be identical or different and are each H or an aliphatic or aromatic hydrocarbon radical having from 1 to 25 carbon atoms and M = H, Li, Na, K or NH₄ and
Z¹, Z² = substituted or unsubstituted, aliphatic or aromatic hydrocarbon radicals having from 1 to 75 carbon atoms, where Z¹ and Z² may be covalently bound to one another.

2. A process according to claim 1,
**characterized in that**
the hydroformylation is carried out in the presence of a ligand of the formula II 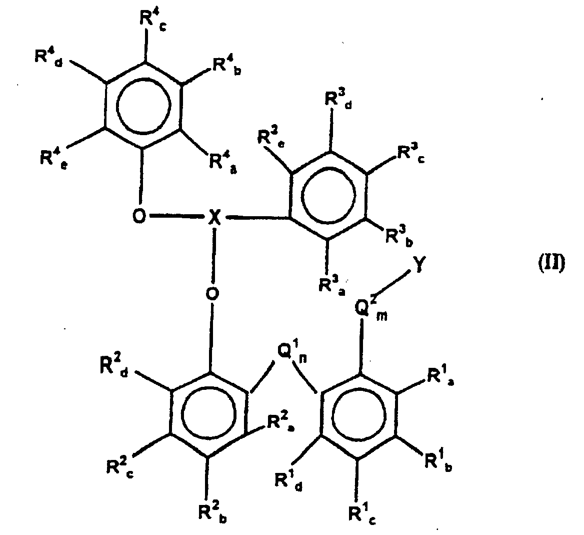 where
X = As, Sb, P,
R¹_{a-d}, R²_{a-d}, R³_{a-c}, R⁴_{a-c} = H, aliphatic or aromatic hydrocarbon radicals, aliphatic or aromatic alkoxy groups, each having from 1 to 25 carbon atoms, where R¹_{a-d}, R²_{a-d}, R³ₐ₋ₑ and R⁴ₐ₋ₑ may in each case be identical or different,
Q¹, Q², = O, S, NR⁷, CR⁷R⁸, where R⁷ and R⁸ may be identical or different and may be as defined for R¹ₐ,
n, m, = 0 or 1 and
Y = -O-R⁵, -COOR⁵, -COOM, -SR⁵, -NR⁵R⁶, -N=CR⁵R⁶, -COR⁵, -CONR⁵R⁶, -F, -Cl, -Br, -I,
where R⁵ and R⁶ may be identical or different and are each H or an aliphatic or aromatic hydrocarbon radical having from 1 to 25 carbon atoms and M = H, Li, Na, K or NH₄.

3. A process according to claim 1,
**characterized in that**
the hydroformylation is carried out in the presence of a ligand of the formula III 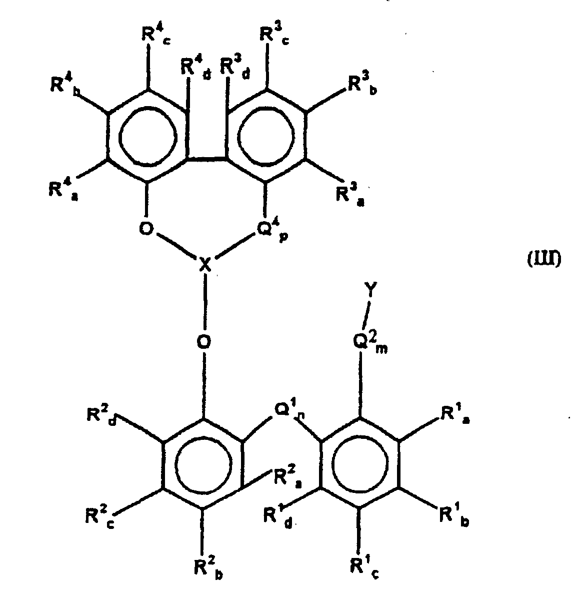 where
X = As, Sb, P,
R¹_{a-d}, R²_{a-d}, R³_{a-d}, R⁴_{a-d} = H, aliphatic or aromatic hydrocarbon radicals, aliphatic or aromatic alkoxy groups, each having from 1 to 25 carbon atoms, where R¹_{a-d}, R²_{a-d}, R³_{a-d}, R⁴_{a-d} may in each case be identical or different,
Q¹, Q², = O, S, NR⁷, CR⁷R⁸, where R⁷ and R⁸ may be identical or different and may be as defined for R¹ₐ,
Q⁴ = CR⁷R⁸, where R⁷ and R⁸ may be identical or different and may be as defined for R¹ₐ,
n, m, p = 0 or 1,
Y = -O-R⁵, -COOR⁵, -COOM, -SR⁵, -NR⁵R⁶, -N=CR⁵R⁶, -COR⁵, -CONR⁵R⁶, -F, -Cl, -Br, -I,
where R⁵ and R⁶ may be identical or different and are each H or an aliphatic or aromatic hydrocarbon radical having from 1 to 25 carbon atoms and M = H, Li, Na, K or NH₄.

4. A process according to claim 1,
**characterized in that**
the hydroformylation is carried out in the presence of a ligand of the formula IV 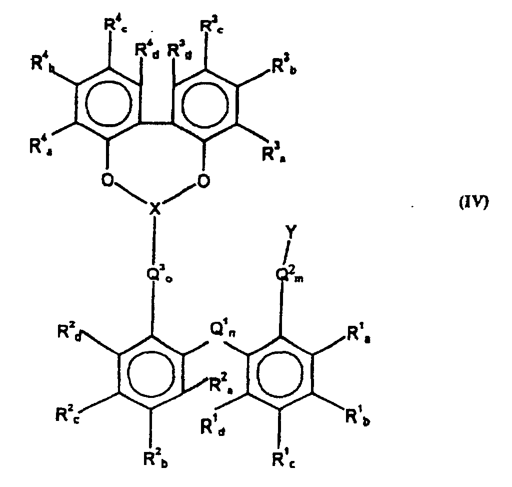 where
X = As, Sb, P,
R¹_{a-d}, R²_{a-d}, R³_{a-d}, R⁴_{a-d} = H, aliphatic or aromatic hydrocarbon radicals, aliphatic or aromatic alkoxy groups, each having from 1 to 25 carbon atoms, where R¹_{a-d}, R²_{a-d}, R³_{a-d} and R⁴_{a-d} may in each case be identical or different,
Q¹, Q², = O, S, NR⁷, CR⁷R⁸, where R⁷ and R⁸ may be identical or different and may be as defined for R¹ₐ,
Q³ = CR⁷R⁸, where R⁷ and R⁸ may be identical or different and may be as defined for R¹ₐ,
n, m, o, = 0 or 1,
Y = -O-R⁵, -COOR⁵, -COOM, -SR⁵, -NR⁵R⁶, -N=CR⁵R⁶, -COR⁵, -CONR⁵R⁶, -F, -Cl, -Br, -I,
where R⁵ and R⁶ may be identical or different and are each H or an aliphatic or aromatic hydrocarbon radical. having from 1 to 25 carbon atoms and M = H, Li, Na, K or NH₄.

5. A process according to any one of claims 1 to 4,
**characterized in that**
the metal of transition group VIII of the Periodic Table is cobalt or rhodium.

6. A process according to any one of claims 1 to 5,
**characterized in that**
olefins having from 3 to 8 carbon atoms are used.

## Revendications

1. Procédé d'hydroformylation catalytique d'oléfines comportant 3 à 24 atomes de carbone.
**caractérisé en ce qu'**on utilise comme catalyseur un métal du groupe secondaire 8 du système périodique en présence d'un coordinat répondant à la formule I 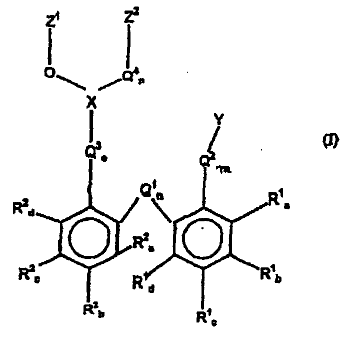 dans laquelle :
X = As, Sb, P,
R¹_{a-d}, R²_{a-d} = H, reste hydrocarboné aliphatique ou aromatique, groupe alcoxy aliphatique ou aromatique, comportant à chaque fois 1 à 25 atomes de carbone, R¹_{a-d} et R²_{a-d} pouvant à chaque fois être identiques ou différents,
Q¹, Q², Q³, Q⁴ = O, S, NR7, CR⁷R⁸, R⁷ et R⁸ pouvant être identiques ou différents et posséder une des significations de R¹ₐ, avec la précision que soit Q³ soit Q⁴ représente O, S, NR⁷.
n, m, o, p = 0 ou 1, avec la précision que o ou p est égal à 1.
Y = -O-R⁵, -COOR⁵, -COOM, -SR⁶, -NR⁵R⁶, -N=CR⁵R⁶, -COR⁵, -CONR⁵R⁶, -F, -Cl, -Br, -I, R⁵ et R⁶ pouvant être identiques ou différents et représenter H, un reste hydrocarboné aliphatique ou aromatique comportant 1 à 25 atomes de carbone et M = H, Li, Na, K ou NH₄, et
Z¹, Z² = reste hydrocarboné aliphatique ou aromatique, substitué ou non substitué, comportant 1 à 75 atomes de carbone, Z¹ et Z² pouvant présenter une liaison covalente.

2. Procédé selon la revendication 1,
**caractérisé en ce que**
l'hydroformylation est effectuée en présence d'un coordinat répondant à la formule II 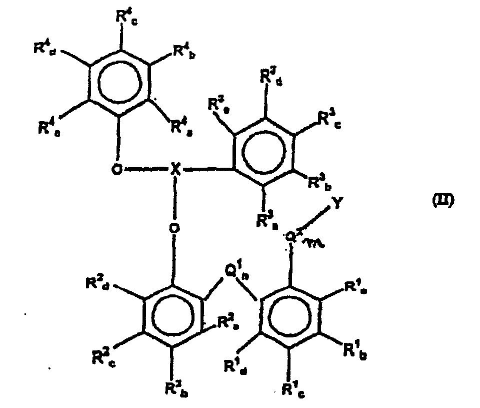 dans laquelle :
X = As ; Sb, P,
R¹_{a-d}, R²_{a-d}, R³_{a-c}, R⁴_{a-c} = H, reste hydrocarboné aliphatique ou aromatique, groupe alcoxy aliphatique ou aromatique, comportant à chaque fois 1 à 25 atomes de carbone, R¹_{a-d} R²_{a-d} R³_{a-c}, et R⁴ₐ₋ₑ pouvant à chaque fois être identiques ou différents,
Q¹, Q², = O, S, NR⁷, CR⁷R⁸, R⁷ et R⁸ pouvant être identiques ou différents et posséder une des significations de R¹ₐ,
n, m, = O ou 1, et
Y = - O-R⁵, -COOR⁵, -COOM, -SR⁵, -NR⁵R⁶, -N = CR⁵R⁶, COR⁵, -CONR⁵R⁶, -F, -Cl, -Br, -I, R⁵ et R⁶ pouvant être identiques ou différents et représenter H, un reste hydrocarboné aliphatique ou aromatique comportant 1 à 25 atomes de carbone et M = H, Li, Na, K ou NH₄.

3. Procédé selon la revendication 1,
**caractérisé en ce que**
l'hydroformylation est effectuée en présence d'un coordinat répondant à la formule III 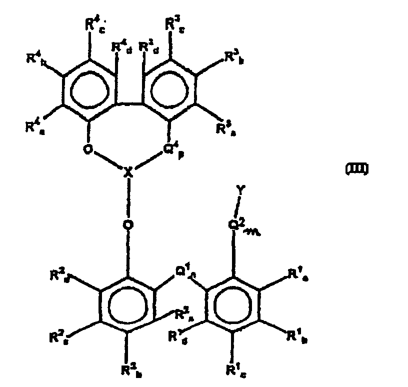 dans laquelle,
X = As, Sb, P,
R¹_{a-d}, R²_{a-d}, R³_{a-d}, R⁴_{a-d} = H, reste hydrocarboné aliphatique ou aromatique, groupe alcoxy aliphatique ou aromatique, comportant à chaque fois 1 à 25 atomes de carbone, R¹_{a-d}, R²_{a-d} R³_{a-d} et R⁴_{a-d} pouvant être à chaque fois être identiques ou différents,
Q¹, Q², = O, S, NR⁷, CR⁷R⁸, R7 et R⁸ pouvant être identiques ou différents et posséder une des significations de R¹ₐ.
Q⁴ = CR⁷R⁸, R⁷ et R⁸ pouvant être identiques ou différents et pouvant posséder une des significations de R¹ₐ,
n, m, p = 0 ou 1,
Y = -O-R⁵, -COOR⁵, -COOM, -SR⁵, -NR⁵R⁶, -N = CR⁵R⁶, -COR⁵, -CONR⁵R⁶, -F, -Cl, -Br, -I, R⁵ et R⁶ pouvant être identiques ou différents et représenter H, un reste hydrocarboné aliphatique ou aromatique comportant 1 à 25 atomes de carbone et M = H, Li, Na, K ou NH₄.

4. Procédé selon la revendication 1,
**caractérisé en ce que**
l'hydroformylation est effectuée en présence d'un coordinat répondant à la formule IV 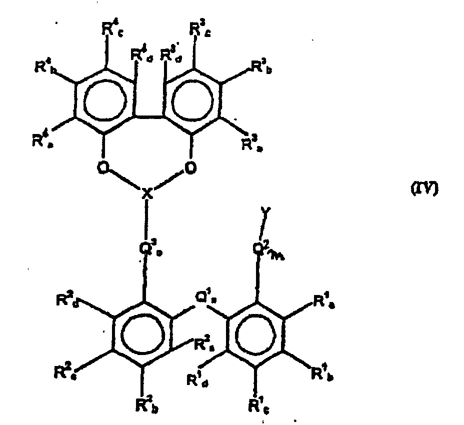 dans laquelle
X = As, Sb, P,
R¹_{a-d}, R²_{a-d}, R³_{a-d}, R⁴_{a-d} = H, reste hydrocarboné aliphatique ou aromatique, groupe alcoxy aliphatique ou aromatique, comportant à chaque fois 1 à 25 atomes de carbone, R¹_{a-d}, R²_{a-d},R³_{a-d} et R⁴_{a-d} pouvant être à chaque fois identiques ou différents,
Q¹, Q², = O, S, NR⁷, CR⁷R⁸, R⁷ et R⁸ pouvant être identiques ou différents et posséder une des significations de R¹ₐ,
Q³ = CR⁷R⁸, R⁷ et R⁸ pouvant être identiques ou différents et posséder une des significations de R¹ₐ,
n, m, o = 0 ou 1,
Y = -O-R⁵, -COOR⁵, -COOM, -SR⁵, -NR⁵R⁶, -N = CR⁵R⁶, -COR⁵, -CONR⁵R⁶, -F, -Cl, -Br, -I, R⁵ et R⁶ pouvant être identiques ou différents et représenter H, un reste hydrocarboné aliphatique ou aromatique comportant 1 à 25 atomes de carbone et M = H, Li, Na, K ou NH₄.

5. Procédé selon l'une quelconque des revendications 1 à 4,
**caractérisé en ce qu'**
on utilise comme métal du groupe secondaire 8 du système périodique, le cobalt ou le rhodium.

6. Procédé selon l'une quelconque des revendications 1 à 5,
**caractérisé en ce qu'**
on utilise des oléfines comportant 3 à 8 atomes de carbone.
